# EUROPÄISCHE PATENTSCHRIFT

(11) **EP 3 243 454 B1**
(45) Veröffentlichungstag und Bekanntmachung des Hinweises auf die Patenterteilung: **07.08.2019**
(21) Anmeldenummer: 17164693.8
(22) Anmeldetag: 04.04.2017
(51) Int. Cl.: A61B 17/56, A61F 2/32

(54) **AM BECKENKNOCHEN MONTIERBARE ABSTÜTZVORRICHTUNG**
SUPPORTING DEVICE MOUNTABLE AT THE PELVIC BONE
DISPOSITIF DE SUPPORT À MONTER SUR L'OS DE LA HANCHE

(30) Priorität: 04.04.2016 EP 16163669
(43) Veröffentlichungstag der Anmeldung: 15.11.2017
(73) Patentinhaber: Baumgart, Rainer, 80539 München (DE)
(72) Erfinder: Baumgart, Rainer, 80539 München (DE)
(74) Vertreter: Lambacher, Michael

(56) Entgegenhaltungen:
- US-A1- 2011 264 216
- US-A1- 2012 253 414
- US-A1- 2014 257 501

## Beschreibung

Die Erfindung betrifft eine am Beckenknochen montierbare Abstützvorrichtung sowie eine die Abstützvorrichtung aufweisende Vorrichtung zur Dehnung von Weichteilen im Hüftbereich.

Aus der US 2012/0253414 A1 ist eine Entlastungsvorrichtung für ein Gelenk bekannt. Gemäß einer Ausführungsform umfasst die Vorrichtung eine Basis und einen über ein Kugelgelenk mit der Basis beweglich gekoppelten starren Stab. Die Vorrichtung ist außerhalb des noch erhaltenen Gelenkes angebracht.

Der endoprothetische Hüftgelenksersatz ist ein verbreitetes operatives Verfahren, wenn das menschliche Hüftgelenk verschlissen ist und starke Schmerzen resultieren. Hierbei wird an der gleichen Stelle, wo das natürliche Hüftgelenk platziert ist, eine Schale implantiert, in der ein künstlicher, runder Gelenkkopf, der mit einem Schaft in dem Oberschenkelknochen verankert ist, artikulieren kann. Derartige Prothesen sind aus der US 2,947,308 A und der DE 295 13 694 U1 bekannt.

Wenn aufgrund einer fehl angelegten (dysplastischen) Pfanne oder nach einem Unfallereignis oder nach einer Tumorresektion das Hüftgelenk kopfwärts (nach cranial) verschoben ist, resultiert regelhaft einerseits eine Beinverkürzung und andererseits meist eine deutliche Bewegungseinschränkung, wobei sich um den Hüftkopf meist ein sogenanntes Neo-Gelenk ausbildet, das eine belastungsfähige Abstützung gewährleistet.

Bei einer derartigen Ausgangssituation, in der das Hüftgelenk kopfwärts ausgewandert ist, ist es bisher nur sehr unbefriedigend möglich, ein künstliches Hüftgelenk zu implantieren, da einerseits die Implantation eines Kunstgelenkes im Bereich der Beckenschaufel sehr schlechte Langzeitergebnisse liefert und andererseits die Platzierung des Kunstgelenkes in anatomisch regelrechter Position nur möglich ist, wenn der Oberschenkelknochen fußwärts gezogen und damit die Weichteile gedehnt werden oder eine Verkürzung des Oberschenkelknochens vorgenommen wird. Eine akute Dehnung von mehr als etwa 2 cm führt regelhaft zu einer Schädigung des Nervus Ischiadikus und damit zu Funktionsausfällen. Bei einer Kürzung des Oberschenkelknochens verbleibt meist eine erhebliche Beinlängendifferenz und zudem eine Funktionsstörung, da wichtige Muskelansätze geopfert werden müssen.

Kontinuierliche Verlängerungen, die das Risiko einer Nervenschädigung verringern, waren früher nur mit externen Fixateuren, die zum einen am Becken und zum anderen am Oberschenkelknochen befestigt werden, möglich. Diese Fixateure sind jedoch extrem belastend für den Patienten und es besteht ein erhebliches Infektions- oder zumindest Kontaminationsrisiko, was im Hinblick auf die darauf folgende Hüftendoprothese besonders problematisch ist.

Es ist bekannt, Beinverlängerungen mit voll-implantierbaren, motorisierten Distraktionsmarknägeln durchzuführen, wie sie beispielsweise in der EP 1 371 346 A1 beschrieben sind. Ein weiteres Anwendungsgebiet dieser Distraktionsmarknägel ist es nun, nicht nur einen Knochen zu verlängern, sondern auch die Weichteile zu dehnen, um z. B. wie in der zuvor geschilderten Situation den Oberschenkelknochen fußwärts zu verlagern, um anschließend eine Hüftgelenksendoprothese in regelrechter Position implantieren zu können.

Erste klinische Anwendungen hierzu, die in "J Bone Joint Surg Vol.87-B, No.4, April 2005" beschrieben sind, gab es bereits vor über 10 Jahren. Die bei dieser Anwendung verwendete Abstützvorrichtung umfasst eine Aufnahmeeinrichtung, die einen Schlitz aufweist, in den das Stützende des Teleskopteils des Distraktionsmarknagels eingeführt wurde, wobei die Bewegung des Stützendes stark eingeschränkt und hinsichtlich der Hebelwirkungen unkontrolliert stattfindet.

Das Problem liegt somit in der Abstützung des Stützendes des Distraktionsmarknagels auf der proximalen beckenzugewandten Seite, da hier einerseits ausreichend Axialkräfte aufgenommen werden müssen, andererseits aber der Bewegungsumfang so wenig wie möglich eingeschränkt sein darf, da ansonsten durch die Hebelkräfte des Beines die Befestigung der Vorrichtung ausbrechen würde.

Die der Erfindung zugrunde liegende Aufgabe liegt nun darin, eine Abstützvorrichtung zu realisieren, die den anatomischen Gegebenheiten an der Außenseite des Beckenknochens angepasst ist und im unteren Bereich, wo genügend Knochenmasse am Pfannendach vorliegt, so befestigt wird, dass die hier auftretenden Zug- und Scherkräfte aufgenommen werden, während die resultierenden Druckkräfte im oberen Bereich flächig übertragen werden. Weiterhin gilt es einerseits, eine weitestgehende Bewegungsfreiheit des Beines zu ermöglichen, damit die Hebelkräfte aufgrund der Länge des Beines (ca. 1 m) die Fixierungsschrauben der Vorrichtung nicht aus dem Beckenknochen ziehen und damit zu einer Lockerung führen und andererseits keine Luxation (Entfernen der Gelenkpartner voneinander) eintritt.

Die der Erfindung zugrunde liegende Aufgabe wird durch eine Vorrichtung zur Dehnung von Weichteilen im Hüftbereich mit einem Distraktionsmarknagel und einer Abstützvorrichtung mit den Merkmalen des Patentanspruchs 1 oder 2 gelöst. Vorteilhafte Ausführungsformen der erfindungsgemäßen Vorrichtung sind Gegenstand der Patentansprüche 3 bis 9.

Bei der erfindungsgemäßen am Beckenknochen montierbaren Abstützvorrichtung umfasst die Aufnahmeeinrichtung einen Kugelsitz mit einer eine Kugelsitzfläche bildenden Kavität zur Aufnahme einer an dem Stützende des Teleskopteils oder des Verankerungsteils vorgesehenen Kugel. Der Innenradius der Kugelsitzfläche entspricht dem Außenradius der Kugel. Der Kugelsitz ist so ausgebildet und angeordnet, dass der Teleskopteil bzw. der Verankerungsteil eine für ein weitgehend normales Bewegungsmuster des Oberschenkelknochens ausreichende Bewegung durchführen und nicht aus dem Kugelsitz luxieren kann, wenn der Teleskopteil bzw. der Verankerungsteil in dem Oberschenkelknochen implantiert ist.

Die ausreichende Bewegung wird beispielsweise gewährleistet, wenn sich die Längsachse des Teleskopteils bzw. des Verankerungsteils innerhalb eines Bereichs eines gedachten Kegels mit einem Kegelwinkel von ca. 25-45°, vorzugsweise ca. 35° bewegen kann, wenn der Verankerungsteil bzw. der Teleskopteil im Oberschenkelknochen implantiert ist und die Abstützvorrichtung am Beckenknochen montiert ist.

Vorzugsweise sind in der Grundplatte bezüglich der Aufnahmeeinrichtung distal wenigstens zwei im Abstand zueinander angeordnete Durchgangsöffnungen vorgesehen. An diesen Durchgangsöffnungen wird die Abstützvorrichtung an dem Beckenknochen mittels Spongiosaschrauben befestigt. Diese Befestigung ist ausreichend, um Zugkräfte aufzunehmen zu können und Druckkräfte auf den Beckenknochen zu übertragen. Befestigungsöffnungen proximal der Aufnahmeeinrichtung sind somit nicht erforderlich, sie können aber zur weiteren Stabilisierung vorgesehen werden. Der proximale Rand der Grundplatte kann unter die an dem Beckenknochen anliegende Muskulatur geschoben werden, ohne dass hier eine zusätzliche Befestigung erforderlich ist, da in diesem Bereich in erster Linie Druckkräfte auftreten.

Bei einer bevorzugten Ausführungsform umfasst der Kugelsitz eine bevorzugt von einem Stützblock gebildete Basis mit einer Teilkavität mit einer ersten Teilkugelsitzfläche, die so ausgebildet und angeordnet ist, dass die Kugel frei in die Teilkavität eingelegt werden kann. Eine Abdeckung mit einer zweiten geringen Teilkugelsitzfläche ist so befestigbar, dass die erste Teilkugelsitzfläche zur Bildung einer Gesamtkugelsitzfläche in die zweite Teilkugelsitzfläche übergeht, wobei die Gesamtkugelsitzfläche größer ist als die Halbkugelaußenfläche der Kugel. Die Abdeckung bildet hierdurch eine Hinterschneidung, die eine Luxation der Kugel aus der Kavität des Kugelsitzes verhindert.

Eine ausreichende Sicherheit ist in diesem Fall gewährleistet, wenn die Gesamtkugelsitzfläche 2% bis 5% größer ist als die Halbkugelaußenfläche.

Wenigstens muss der Innenumfang der Gesamtkugelsitzfläche an einer Stelle größer sein als der halbe Umfang der Kugel.

Wenn die Grundplatte an ihrem proximalen Rand abgeflacht ist, kann der proximale Rand der Grundplatte ohne Verletzungsgefahr unter die Muskulatur am Beckenknochen geschoben werden.

Zur Verminderung der Gleitreibung wird bevorzugt eine Kunststoffschale in die Kavität eingesetzt oder eine reibungsvermindernde Beschichtung auf der Kugel aufgebracht oder die Kavität mit reibungsverminderndem Material beschichtet.

Ausführungsbeispiele der Erfindung werden nachstehend anhand von Zeichnungen näher erläutert. Es zeigen:
Figur 1 eine perspektivische Ansicht einer erfindungsgemäßen Abstützvorrichtung;
Figur 2 eine Draufsicht auf die Abstützvorrichtung von Fig. 1;
Figur 3 einen medianen Querschnitt der Abstützvorrichtung von Fig. 1;
Figur 4 eine die Abstützvorrichtung aufweisende Vorrichtung zur Dehnung von Weichteilen im implantierten Zustand zu Beginn einer Behandlung;
Figur 5 die Vorrichtung von Figur 4 nach Dehnung der Weichteile gegen Ende einer Behandlung;
Figur 6 eine weitere, die Abstützvorrichtung aufweisende Vorrichtung zur Dehnung von Weichteilen im implantierten Zustand zu Beginn einer Behandlung;
Figur 7 die Vorrichtung von Figur 6 nach Dehnung der Weichteile gegen Ende der Behandlung; und
Figur 8 einen Teilabschnitt eines Distraktionsmarknagels mit einem Zwischenstück zur beweglichen Aufnahme in der Abstützvorrichtung von Fig. 1.

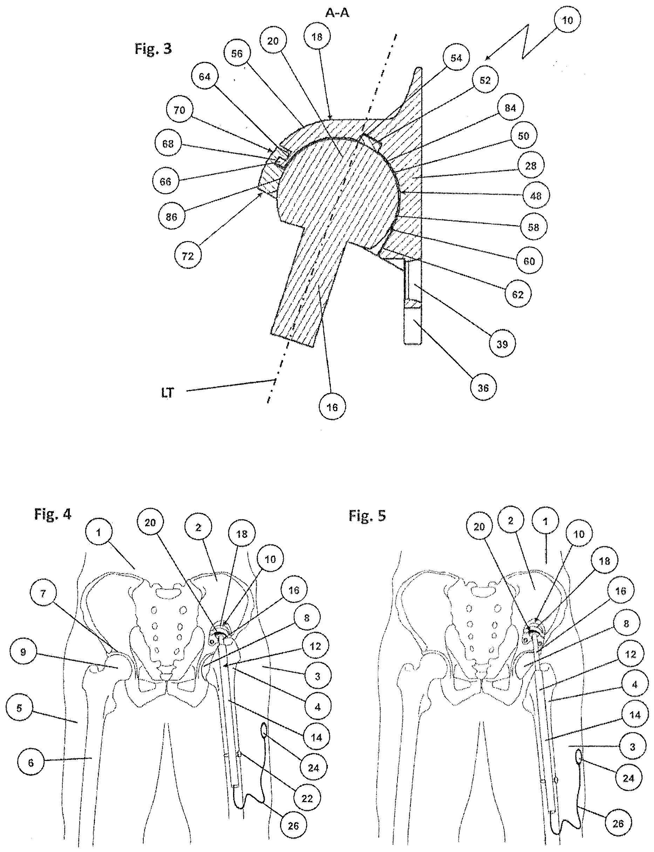

Zunächst zu den Figuren 4 und 5. In den Figuren 4 und 5 ist schematisch der Skelettaufbau eines Patienten im Hüftbereich gezeigt, wobei in Figur 4 der linke Oberschenkel des Patienten kopfwärts (nach cranial) verschoben ist. Diese Verschiebung kann aus einer fehl angelegten Pfanne oder nach einem Unfallereignis oder nach einer Tumorresektion resultieren.

Im unteren Bereich des Torsos 1 ist das Becken 2 angeordnet. In Figur 4 ist der Gelenkkopf 9 am oberen Ende des Oberschenkelknochens 6 des rechten Oberschenkels 5 korrekt in der Hüftpfanne 7 angeordnet. Bei dem linken Oberschenkel 3 des Patienten ist der Oberschenkelknochen 4 bezüglich der Hüftpfanne 8 kopfwärts nach oben verschoben.

Um eine Verlagerung des Oberschenkelknochens 4 fußwärts zu ermöglichen und die entsprechenden Weichteile zu dehnen, ist in dem Oberschenkelknochen 4 ein Distraktionsmarknagel 12 implantiert, der einen in Längsrichtung des Oberschenkelknochens 4 in diesem implantierten Verankerungsteil 14 umfasst, der mittels wenigstens einer quer angeordneten Befestigungsschraube 22 an dem Oberschenkelknochen 4 befestigt ist. In dem Verankerungsteil 14 ist ein Teleskopteil 16 in Längsrichtung des Verankerungsteils 14 verschiebbar geführt. An dem proximalen, d.h. dem Torso 1 zugewandten Ende des Teleskopteils 16 ist eine Kugel 20 beispielsweise durch Kegelpresssitz befestigt, die in einer Aufnahmeeinrichtung 18 einer Abstützvorrichtung 10 gelenkig gelagert ist. Die Abstützvorrichtung 10 ist am Os Ilium des Beckens 2 montiert. Der Teleskopteil 16 kann mittels eines in dem Verankerungsteil 14 angeordneten Motors in proximaler Richtung verschoben werden. Zur Steuerung des Motors ist der Motor über ein Kabel 26 mit einer unter der Haut des Oberschenkels angeordneten Antenne 24 verbunden. Eine entsprechende Konstruktion eines Marknagels ist beispielsweise aus der EP 1 371 346 A1 bekannt.

Der Teleskopteil 16 wird schrittweise über einen längeren Zeitraum durch den Motor in proximaler Richtung verschoben. Der Teleskopteil 16 stützt sich dabei über die am proximalen Stützende des Teleskopteils 16 angeordnete Kugel 20, die in der Aufnahmeeinrichtung 18 gelenkig aufgenommen ist, an der Abstützeinrichtung 10 ab, die wiederum an dem Os Ilium des Beckens 2 montiert ist. Hierdurch wird der Oberschenkelknochen 4 in distaler Richtung, d.h. vom Torso 1 weg, verschoben, wobei gleichzeitig die Weichteile gedehnt werden. Ein solches Verfahren zur Dehnung der Weichteile ist in der Druckschrift "J Bone Joint Surg Vol.87-B, No.4, April 2005" beschrieben, so dass das Verfahren im Folgenden nicht weiter erläutert wird.

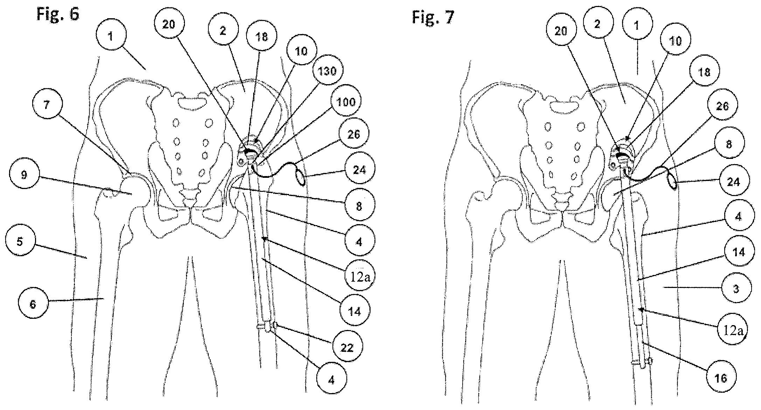

In Zusammenhang mit den Figuren 6 und 7 wird eine weitere Vorrichtung zur Dehnung von Weichteilen beschrieben. Es sei angemerkt, dass der in den Figuren 6 und 7 dargestellte Skelettaufbau mit einem nach oben verschobenen linken Oberschenkel dem in den Figuren 4 und 5 dargestellten Skelettaufbau entspricht und mit denselben Bezugszeichen versehen ist. Um Wiederholungen zu vermeiden sei auf die Beschreibung der Figuren 4 und 5 weiter oben verwiesen.

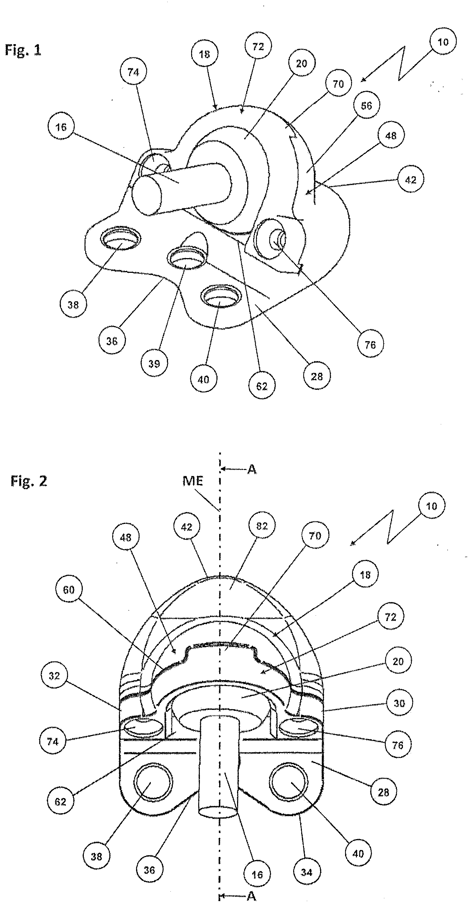

Die Vorrichtung zur Dehnung von Weichteilen umfasst einen im Oberschenkelknochen 4 implantierbaren Distraktionsmarknagel 12a sowie eine Abstützvorrichtung 10 zur proximalen Abstützung des Distraktionsmarknagels 12a. Die Abstützvorrichtung 10 ist baugleich zu der in den Figuren 4 und 5 gezeigten Abstützvorrichtung und wird in Zusammenhang mit den Figuren 1 bis 3 weiter unten im Detail beschrieben.

Der Distraktionsmarknagel 12a umfasst wiederum einen Verankerungsteil 14, einen im Verankerungsteil 14 verschiebbar aufgenommen Teleskopteil 16 sowie einen im Verankerungsteil 14 angeordneten Antrieb zur linearen Verschiebung des Teleskopteils 16 relativ zum Verankerungsteil 14. Der Antrieb umfasst einen Motor und eine Getriebeeinheit. Diese sind in den in den Figuren 6 und 7 nicht dargestellt. Durch Betätigung des Antriebs kann der Teleskopteil 16 in Längsrichtung des Verankerungsteils 14 ein- und ausgefahren werden. Die Steuerung des Motors erfolgt wiederum über ein Kabel 26 und einer unter der Haut des Oberschenkels angeordneten Antenne 24.

Der Distraktionsmarknagel 12a umfasst somit dieselben Komponenten wie der Distraktionsmarknagel 12 in den Figuren 4 und 5 und unterscheidet sich von diesem darin, dass die Kugel 20 zur Lagerung an der Abstützvorrichtung 10 an dem dem Teleskopteil 16 gegenüberliegenden Ende des Verankerungsteils 14 angeordnet ist. Entsprechend unterscheidet sich die in den Figuren 6 und 7 gezeigte Vorrichtung zur Dehnung von Weichteilen von jener in den Figuren 4 und 5 gezeigten Vorrichtung darin, dass der Distraktionsmarknagel 12a über seinen Verankerungsteil 14 an der Abstützvorrichtung 10 beweglich gelagert ist und der Teleskopteil 16 mittels einer quer angeordneten Befestigungsschraube 22 am Oberschenkelknochen 4 befestigbar ist. Der Distraktionsmarknagel 12a in den Figuren 6 und 7 ist somit, verglichen zur Anordnung in den Figuren 4 und 5, um 180° gedreht angeordnet.
Figur 6 zeigt die Vorrichtung zur Dehnung von Weichteilen im implantierten Zustand zu Beginn einer Behandlung. Der Teleskopteil befindet sich in einer eingefahrenen Position. Lediglich das distale Ende des Teleskopteils 16 ragt aus dem distalen Ende des Verankerungsteils 14 hervor und ist über einen Nagel 22 am Oberschenkelknochen befestigt. Um eine Verlagerung des Oberschenkelknochens 4 fusswärts zu ermöglichen und die entsprechenden Weichteile zu dehnen, kann der Teleskopteil 16 schrittweise mittels des Motors in distale Richtung (also vom Torso 1 weg) verschoben werden. Durch das schrittweise Ausfahren des Teleskopteils 16 wird der Oberschenkelknochen 4 in distaler Richtung verschoben. Die in den Figuren 6 und 7 gezeigte Vorrichtung erreicht somit dasselbe Ergebnis wie die Vorrichtung in den Figuren 4 und 5. Der einzige Unterschied liegt in der Orientierung und Lagerung des Distraktionsmarknagels 12a, welcher in der in den Figuren 6 und 7 gezeigten Ausführung mit seinem Teleskopteil 16 am Oberschenkelknochen 4 befestigt ist und mit einer am proximalen Ende des Verankerungsteils 14 angebrachten Kugel 12 in der Aufnahmeeinrichtung 10 beweglich gelagert ist.

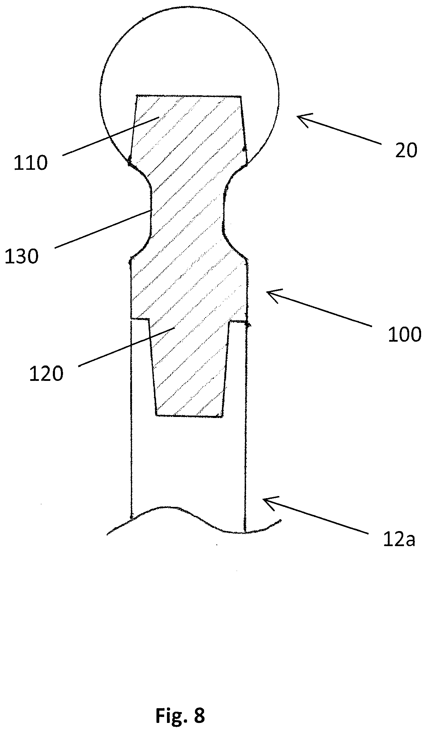

Gemäß einer Implementierung kann der in Zusammenhang mit den Figuren 6 und 7 beschriebene Distraktionsmarknagel 12a ferner ein zwischen der Kugel 20 und dem proximalen Ende des Distraktionsmarknagels 12a angeordnetes Zwischenstück 100 aufweisen. Die Position des Zwischenstücks 100 ist in Fig. 6 angedeutet. Fig. 8 zeigt schematisch das proximale Ende des Distraktionsmarknagels 12a zusammen mit dem Zwischenstück 100 und der Kugel 20.

Das Zwischenstück 100 ist mit seinem distalen Ende 120 am Verankerungsteil 14 des Distraktionsmarknagels 120a befestigt. Hierzu kann das Zwischenstück 100 mit dem proximalen Ende des Verankerungsteils 14 formschlüssig und/oder stoffschlüssig verbunden sein. Am proximalen Ende 110 des Zwischenstückes 100 ist ferner die Kugel 20 zur beweglichen Aufnahme in der Abstützvorrichtung 10 befestigt. Somit bildet das Zwischenstück 100 zusammen mit der Kugel 20 das Stützende des Distraktionsmarknagels 12a. Durch die Verwendung des Zwischenstücks 100 kann somit ein herkömmlicher Distraktionsmarknagel zum Einsatz kommen, um die in Zusammenhang mit den Figuren 6 und 7 beschriebene Vorrichtung zur Dehnung von Weichteilen zu realisieren. Der herkömmliche Distraktionsmarknagel muß lediglich mit dem hier beschriebenen Zwischenstück 100 mit Kugel 20 versehen werden.

Gemäß der in Figur 8 gezeigten Ausführung ist die Kugel 20 am stabförmigen proximalen Ende des Zwischenstücks 100 aufgesetzt und mit diesem fest verbunden. Alternativ ist auch denkbar, dass die Kugel 20 zusammen mit dem Zwischenstück 100 als einstückiges Stützelement ausgebildet ist. Unabhängig von der hier beschriebenen ein- oder zweiteiligen Ausgestaltung kann gemäß einer vorteilhaften Ausführung das Zwischenstück 100 ferner eine in Umfangsrichtung des Zwischenstücks 100 verlaufende Einschnürung 130 aufweisen (siehe Fig. 8), die vom proximalen Ende aus betrachtet (unmittelbar) hinter der Kugel 20 am Zwischenstück 100 angeordnet ist. Durch diese Einschnürung 130 am Zwischenstück 130 wird der Bewegungsumfang des Distraktionsmarknagels relativ zur Abstützvorrichtung 10 weiter erhöht.

Der Aufbau der Abstützvorrichtung 10 und die Lagerung der Kugel 20 des Teleskopteils 16 bzw. des Verankerungsteils 14 in der Aufnahmeeinrichtung 18 ist in den Figuren 1 bis 3 im Detail gezeigt.

Die Abstützvorrichtung 10 ist bezüglich einer senkrecht zur Plattenebene verlaufenden Mittelebene ME symmetrisch ausgebildet. Im Folgenden werden bezüglich der Anordnung einzelner Elemente die Ausdrücke "proximal" und "distal" verwendet, wobei proximal dem Torso 1 zugewandt und distal von dem Torso 1 abgewandt bedeutet.

Die Abstützvorrichtung 10 umfasst eine Grundplatte 28, die zwei Seitenränder 30, 32 aufweist, die im distalen Abschnitt parallel zueinander und parallel zur Mittelebene ME verlaufen und anschließend in proximaler Richtung zur Mittelebene ME hin gekrümmt verlaufen, so dass sie eine abgerundete vordere Spitze 42 am Schnittpunkt mit der Mittelebene ME bilden. In dem distalen, insgesamt senkrecht zur Mittelebene ME verlaufenden Seitenrand 34 ist mittig eine Einbuchtung 36 ausgebildet. Beidseitig der Mittellinie ME gehen angrenzend an den distalen Seitenrand 34 zwei Durchgangsöffnungen 38, 40 durch die Grundplatte 28 hindurch. Eine weitere Durchgangsöffnung 39 ist mittig zwischen den Durchgangsöffnungen 38 und 40 ausgebildet.

Proximal zu den Durchgangsöffnungen 38, 39, 40 ist auf der Grundplatte 28 eine Aufnahmeeinrichtung 18 vorgesehen, die einen Kugelsitz 48 umfasst, in dem eine Kavität 58 ausgebildet ist, in der die Kugel 20 am Stützende des Teleskopteils 16 bzw. am Stützende des Verankerungsteils 14 aufgenommen ist.

Der Kugelsitz 48 besteht aus zwei Elementen, die jeweils eine Teilkugelsitzfläche bilden. Ein Stützblock 56 erstreckt sich von der Oberseite der Grundplatte 28 nach oben. In dem Stützblock 56 ist eine Teilkavität 84 ausgebildet, die eine erste Teilkugelsitzfläche definiert. Die Teilkavität 84 ist in distaler Richtung hin offen und wird durch einen Stirnrand 60 begrenzt, der senkrecht zur Mittelebene ME verläuft.

Wie es in Figur 3 zu erkennen ist, ist der Kugelsektor des sphärischen Abschnittes der Teilkavität 84 etwas kleiner als der Halbkugelsektor der Kugel 20 des Teleskopteils 16 bzw. der Kugel des Verankerungsteils 14. Von dem unteren Ende des sphärischen Teils der Teilkavität 84 erstreckt sich die Oberfläche einer Schrägstufe 62 im Wesentlichen tangential in distaler Richtung schräg nach oben.

Die Teilkavität 84 des Stützblocks 56 ist daher so ausgebildet, dass die Kugel 20 des Teleskopteils 16 bzw. des Verankerungsteils 14 in die Teilkavität 84 eingelegt werden kann, wenn keine Abdeckung 72 angebracht ist, ohne das die Kugel 20 akut nach distal verschoben werden muss.

Die Abdeckung 72 wird an der Stirnseite des Stützblocks 56 angebracht. Die Abdeckung 72 ist im Wesentlichen U-förmig ausgebildet und liegt mit den freien Enden ihrer Schenkel auf der Schrägstufe 62 auf. An den Enden der beiden freien Schenkel ist jeweils eine Durchgangsöffnung 74 und 76 ausgebildet, durch die Befestigungsschrauben hindurchgehen können, die die Abdeckung 72 an dem Stützblock 56 befestigen. Die Innenseite der Abdeckung 72 bildet im oberen, bogenförmigen Bereich eine zweite Teilkugelsitzfläche 86, die bündig an die Teilkugelsitzfläche der Kavität 58 anschließt, wodurch die Gesamtkugelsitzfläche so erhöht wird, dass sie größer ist als die Halbkugelfläche der Kugel 20. Mit anderen Worten wird durch die Abdeckung 72 eine Hinterschneidung gebildet, die verhindert, dass die Kugel 20 aus dem Kugelsitz 48 luxieren kann, wenn die Abdeckung 72 an dem Stützblock 56 angebracht ist.

Für eine feste Verankerung der Abdeckung 72 an dem Stützblock 56 ist im oberen Abschnitt des Stützblocks 56 angrenzend an den Stirnrand 60 eine Nut 64 ausgebildet, wodurch am Rand 60 ein Randvorsprung 66 definiert wird. Gleichzeitig ist in der Abdeckung 72 ein Eingriffsschenkel 70 mit einer Eingriffsnut 68 ausgebildet, die über den Randvorsprung 66 in die Nut 64 eingreift. Durch die Bildung der Eingriffsnut 68 wird somit am proximalen Rand der Abdeckung 72 ein Eingriffsschenkel 70 gebildet, der in die Nut 64 eingreift. Hierdurch wird eine formschlüssige Verbindung der Abdeckung 72 mit dem Stützblock 56 geschaffen, wenn die Abdeckung 72 mittels durch die Durchgangsöffnungen 74, 76 hindurchgehender Befestigungsschrauben an dem Stützblock 56 befestigt wird.

Um die Gleitreibung zwischen der Kugel 20 in der Kavität 58 des Kugelsitzes 48 zu verringern, ist in der Kavität 58 eine Kunststoffschale 50 angeordnet, die an der Sitzfläche anliegt. An der Kunststoffschale 50 ist im proximalen Bereich ein Vorsprung 52 ausgebildet, der in eine Ausnehmung 54 in der Kavität 58 eingreift, wodurch eine sphärische Verschiebung der Kunststoffschale verhindert wird und ein sicherer Halt der Kunststoffschale 50 in der Kavität 58 gewährleistet ist. Die Kunststoffschale 50 bildet somit die tatsächliche Kavität, deren Innendurchmesser dem Außendurchmesser der Kugel 20 entspricht.

Der Kugelsitz 48 ist insgesamt so ausgebildet, dass sich die Längsachse LT des Teleskopteils 16 bzw. des Verankerungsteils 14 innerhalb eines Bereichs eines gedachten Kegels mit einem Kegelwinkel von 25-45°, vorzugsweise 35°, bewegen kann, wenn der Verankerungsteil 14 bzw. der Teleskopteil 16 im Oberschenkelknochen 4 implantiert ist und die Abstützvorrichtung 10 am Beckenknochen 2 montiert ist. Proximal der Aufnahmevorrichtung 18 ist die Grundplatte 28 zur Spitze 42 hin abgeflacht, wodurch die Spitze 42 unter die dem Beckenknochen anliegende Muskulatur ohne Verletzungsgefahr geschoben werden kann.

Insbesondere ist die Grundplatte 28 als flache Platte ausgebildet, welche eine der Aufnahmeeinrichtung 18 gegenüberliegende ebene Plattenseite (also ebene Plattenrückseite) aufweist. Die Abstützvorrichtung 10 kann somit mit der ebenen Rückseite der Grundplatte am Beckenknochen angelegt und befestigt werden. Gleichzeitig ermöglicht die auf der Grundplattenvorderseite angeordnete Aufnahmeeinrichtung 18 eine bewegliche Aufnahme der Kugel 20 derart, dass eine gute Beweglichkeit des Distraktionsmarknagels 12, 12a in Frontal- und Sagittalebene gegeben ist.

Die Grundplatte 28 bildet mit der Aufnahmeeinrichtung 18 bis auf die Abdeckung 72 bevorzugt ein Teil, wobei alle Ränder und Kanten abgerundet sind, um eine Verletzungsgefahr zu minimieren.

Die Grundplatte 28 mit der Aufnahmevorrichtung 18 und der Abdeckung 72 ist vorzugsweise in Stahl oder Titan ausgebildet. Die Kunststoffschale 50 ist vorzugsweise aus PTFE oder PEEK ausgebildet.

### Bezugszeichenliste

1 - Rumpf
2 - Beckenknochen
3 - linker Oberschenkel
4 - linker Oberschenkelknochen
5 - rechter Oberschenkel
6 - rechter Oberschenkelknochen
7 - Hüftpfanne rechts
8 - Hüftpfanne links
9 - Gelenkkopf
10 - Abstützvorrichtung
12, 12a - Distraktionsmarknagel
14 - Verankerungsteil
16 - Teleskopteil
18 - Aufnahmeeinrichtung
20 - Kugel
22 - Schraube
24 - Antenne
26 - Kabel
28 - Grundplatte
30 - rechter Seitenrand
32 - linker Seitenrand
34 - distaler Seitenrand
36 - Einbuchtung
38 - Durchgangsöffnung
39 - Durchgangsöffnung
40 - Durchgangsöffnung
42 - Spitze
48 - Kugelsitz
50 - Kunststoffschale
52 - Vorsprung
54 - Ausnehmung
56 - Stützblock
58 - Kavität
60 - Stirnrand
62 - Schrägstufe
64 - Nut
66 - Randvorsprung
68 - Eingriffsnut
70 - Eingriffsschenkel
72 - Abdeckung
74 - Durchgangsöffnung
76 - Durchgangsöffnung
82 - Abflachung
84 - Teilkavität
86 - Teilkugelsitzfläche
100 - Zwischenstück
110 - Zwischenstück, proximales Ende
120 - Zwischenstück, distales Ende
130 - Einschnürung

## Patentansprüche

1. Vorrichtung zur Dehnung von Weichteilen im Hüftbereich, umfassend:
einen Distraktionsmarknagel (12), der einen in einen Oberschenkelknochen (4) implantierbaren, distalen Verankerungsteil (14) und einen bezüglich des Verankerungsteils (14) in Längsrichtung verfahrbaren, proximalen Teleskopteil (16) mit einem proximalen Stützende aufweist, an dem eine Kugel (20) angebracht ist; und
eine am Beckenknochen (2) montierbare Abstützvorrichtung (10) zur Abstützung des proximalen Stützendes des Distraktionsmarknagels (12), wobei die Abstützvorrichtung (10) eine am Beckenknochen (2) befestigbare Grundplatte (28) und eine auf der Grundplatte (28) vorgesehene Aufnahmeeinrichtung (18) zur beweglichen Aufnahme des Stützendes des Distraktionsmarknagels (12) umfasst, wobei die Aufnahmeeinrichtung (18) einen Kugelsitz (48) mit einer eine Kugelsitzfläche bildenden Kavität (58) zur Aufnahme der an dem proximalen Stützende vorgesehenen Kugel (20) umfasst, wobei der Innenradius der Kugelsitzfläche dem Außenradius der aufnehmbaren Kugel (20) entspricht, und wobei der Kugelsitz (48) so ausgebildet ist, dass sich die Längsachse (LT) des Distraktionsmarknagels (12) innerhalb eines Bereichs eines gedachten Kegels mit einem Kegelwinkel von 25-45°, vorzugsweise ca. 35°, bewegen und nicht aus dem Kugelsitz (48) luxieren kann, wenn der Distraktionsmarknagel (12) im Oberschenkelknochen (4) implantiert ist und die Abstützvorrichtung (10) am Beckenknochen (2) montiert ist.

2. Vorrichtung zur Dehnung von Weichteilen im Hüftbereich, umfassend:
einen Distraktionsmarknagel (12a) mit einem proximalen Verankerungsteil (14) und einem bezüglich des Verankerungsteils (14) in Längsrichtung verfahrbaren, distalen Teleskopteil (16), wobei der Verankerungsteil (14) an seinem dem Teleskopteil (16) gegenüberliegenden proximalen Ende ein Stützende mit einer Kugel (20) aufweist; und
eine am Beckenknochen montierbare Abstützvorrichtung zur Abstützung des proximalen Stützendes des Distraktionsmarknagels (12a),
wobei die Abstützvorrichtung (10) eine am Beckenknochen (2) befestigbare Grundplatte (28) und eine auf der Grundplatte (28) vorgesehene Aufnahmeeinrichtung (18) zur beweglichen Aufnahme des Stützendes des Distraktionsmarknagels (12a) umfasst, wobei die Aufnahmeeinrichtung (18) einen Kugelsitz (48) mit einer eine Kugelsitzfläche bildenden Kavität (58) zur Aufnahme der an dem proximalen Stützende vorgesehenen Kugel (20) umfasst, wobei der Innenradius der Kugelsitzfläche dem Außenradius der aufnehmbaren Kugel (20) entspricht, und wobei der Kugelsitz (48) so ausgebildet ist, dass sich die Längsachse (LT) des Distraktionsmarknagels (12a) innerhalb eines Bereichs eines gedachten Kegels mit einem Kegelwinkel von 25-45°, vorzugsweise ca. 35°, bewegen und nicht aus dem Kugelsitz (48) luxieren kann, wenn der Distraktionsmarknagel (12a) im Oberschenkelknochen (4) implantiert ist und die Abstützvorrichtung (10) am Beckenknochen (2) montiert ist.

3. Vorrichtung nach einem der vorhergehenden Ansprüche, wobei der Distraktionsmarknagel (12, 12a) an seinem Stützende ein Zwischenstück (100) zur Aufnahme der Kugel (20) aufweist.

4. Vorrichtung nach Anspruch 3, wobei das Zwischenstück (100) eine in Umfangsrichtung verlaufende Einschnürung (130) aufweist, die vom proximalen Ende aus betrachtet unmittelbar hinter der Kugel (20) am Zwischenstück (100) angeordnet ist.

5. Vorrichtung nach einem der vorhergehenden Ansprüche, wobei in der Grundplatte (28) bezüglich der Aufnahmeeinrichtung (18) distal wenigstens zwei im Abstand zueinander angeordnete Durchgangsöffnungen (74, 76) vorgesehen sind.

6. Vorrichtung nach einem der vorhergehenden Ansprüche, wobei der Kugelsitz (48) eine Basis mit einer Teilkavität (84) mit einer ersten Teilkugelsitzfläche umfasst, die so ausgebildet und angeordnet ist, dass die Kugel (20) frei in die Teilkavität (84) eingelegt werden kann, und eine Abdeckung (72) mit einer zweiten Teilkugelsitzfläche umfasst, die so befestigbar ist, dass die erste Teilkugelsitzfläche zur Bildung einer Gesamtkugelsitzfläche in die zweite Teilkugelsitzfläche übergeht, wobei die Gesamtkugelsitzfläche größer ist als die Halbkugelaußenfläche der Kugel (20).

7. Vorrichtung nach Anspruch 6, wobei die Gesamtkugelsitzfläche 5% bis 10% größer ist als die Halbkugelaußenfläche.

8. Vorrichtung nach einem der vorhergehenden Ansprüche, wobei die Grundplatte (28) an ihrem proximalen Rand abgeflacht ist.

9. Vorrichtung nach einem der vorhergehenden Ansprüche, wobei zur Verminderung der Gleitreibung eine Kunststoffschale (50) in die Kavität (58) eingesetzt ist oder eine reibungsvermindernde Beschichtung auf der Kugel (20) aufgebracht ist oder die Kavität (58) mit reibungsverminderndem Material beschichtet ist.

## Claims

1. An apparatus for stretching soft tissue parts in the hip region, comprising:
a distraction marrow nail (12) that has a distal anchoring part (14) that can be implanted into a femur (4) and a proximal telescoping part (16) that can be moved in the longitudinal direction with reference to the anchoring part (14) and has a proximal support end, at which a ball (20) is attached, and
a support apparatus (10) that can be mounted on the pelvic bone (2) to support the proximal support end of the distraction marrow nail (12), wherein the support apparatus (10) comprises a base plate (28) that can be affixed to the pelvic bone (2) and an accommodating device (18) that is provided on the base plate (28) for movably accommodating the support end of the distraction marrow nail (12), wherein the accommodating device (18) comprises a ball seat (48) having a cavity (58) that forms a ball seat surface for accommodating a ball (20) provided at the proximal support end, wherein the inside radius of the ball seat surface corresponds to the outside radius of the ball (20) to be accommodated, and wherein the ball seat (48) is configured in such a manner that the longitudinal axis (LT) of the distraction marrow nail (12) can move within a range of an imaginary cone having a cone angle of 25-45°, preferably approximately 35°, and cannot luxate out of the ball seat (48) when the distraction marrow nail (12) is implanted in the femur (4) and the support apparatus (10) is mounted on the pelvic bone (2).

2. An apparatus for stretching soft tissue parts in the hip region, comprising:
a distraction marrow nail (12a) that has a proximal anchoring part (14) and a distal telescoping part (16) that can be moved in the longitudinal direction with reference to the anchoring part (14), wherein the anchoring part (14) at its proximal end located opposite the telescoping part (16) comprises a support end having a ball (20); and
a support apparatus that can be mounted on the pelvic bone to support the proximal support end of the distraction marrow nail (12a),
wherein the support apparatus (10) comprises a base plate (28) that can be affixed to the pelvic bone (2) and an accommodating device (18) that is provided on the base plate (28) for movably accommodating the support end of the distraction marrow nail (12a), wherein the accommodating device (18) comprises a ball seat (48) having a cavity (58) that forms a ball seat surface for accommodating a ball (20) provided at the proximal support end, wherein the inside radius of the ball seat surface corresponds to the outside radius of the ball (20) to be accommodated, and wherein the ball seat (48) is configured in such a manner that the longitudinal axis (LT) of the distraction marrow nail (12a) can move within a range of an imaginary cone having a cone angle of 25-45°, preferably approximately 35°, and cannot luxate out of the ball seat (48) when the distraction marrow nail (12, 12a) is implanted in the femur (4) and the support apparatus (10) is mounted on the pelvic bone (2).

3. The apparatus according to one of the preceding claims, wherein the distraction marrow nail (12, 12a) at the support end thereof has an intermediate piece (100) for accommodating the ball (20).

4. The apparatus according to claim 3, wherein the intermediate piece (100) comprises a constriction (130) extending in the circumferential direction, which is disposed directly behind the ball (20) on the intermediate piece (100), when viewed from the proximal end.

5. The apparatus according to one of the preceding claims, wherein at least two passage openings (74, 76) disposed at a distance from one another are provided in the base plate (28) distally with reference to the accommodating device (18).

6. The apparatus according to one of the preceding claims, wherein the ball seat (48) comprises a base having a sub-cavity (84) with a first partial ball seat surface, which is configured and disposed in such a manner that the ball (20) can be freely laid into the sub-cavity (84), and comprises a covering (72) having a second partial ball seat surface, which can be affixed in such a manner that the first partial ball seat surface makes a transition into the second partial ball seat surface to form a total ball seat surface, wherein the total ball seat surface is larger than the external hemispherical surface of the ball (20).

7. The apparatus according to claim 6, wherein the total ball seat surface is 5% to 10% larger than the external hemispherical surface.

8. The apparatus according to one of the preceding claims, wherein the base plate (28) is flattened at its proximal edge.

9. The apparatus according to one of the preceding claims, wherein in order to reduce the slide friction, a plastic shell (50) is inserted into the cavity (58) or a friction-reducing coating is applied to the ball (20) or the cavity (58) is coated with friction-reducing material.

## Revendications

1. Dispositif pour allonger des parties molles dans la région de la hanche, comprenant :
un clou médullaire de distraction (12), qui comporte une pièce d'ancrage (14) distale, implantable dans un os de fémur (4) et une pièce télescopique (16) proximale pouvant se déplacer dans le sens longitudinal par rapport à la partie d'ancrage (14) et dotée d'une extrémité d'appui proximale, sur laquelle une tête sphérique (20) est disposée,
un dispositif de support (10) à monter sur l'os du bassin (2) destiné à supporter l'extrémité d'appui proximale du clou médullaire de distraction (12), dans lequel le dispositif de support (10) comprend une embase (28) pouvant être fixée à l'os du bassin (2) et un dispositif de logement (18) agencé sur l'embase (28) pour recevoir de façon mobile l'extrémité d'appui du clou médullaire de distraction (12), et le dispositif de logement (18) comprend un logement sphérique (48) comportant une cavité (58) formant une surface de logement sphérique destinée à recevoir la tête sphérique (20) disposée sur l'extrémité d'appui proximale, dans lequel le rayon intérieur de la surface de logement sphérique correspond au rayon extérieur de la tête sphérique (20) pouvant être logée et dans lequel le logement sphérique (48) est configuré de manière à ce que l'axe longitudinal (LT) du clou médullaire de distraction (12) puisse se déplacer dans une région d'un cône imaginaire ayant un angle de cône de 25 à 45°, de préférence d'environ 35° et ne puisse pas se déboîter hors du logement sphérique (48) lorsque le clou médullaire de distraction (12) est implanté dans l'os du fémur (4) et lorsque le dispositif de support (10) est monté sur l'os du bassin (2).

2. Dispositif pour allonger des parties molles dans la région de la hanche, comprenant :
un clou médullaire de distraction (12a) comportant une pièce d'ancrage (14) proximale et une pièce télescopique (16) distale pouvant se déplacer dans le sens longitudinal par rapport à la partie d'ancrage (14), dans lequel la pièce d'ancrage (14) comprend à son extrémité proximale opposée à la pièce télescopique (16) une extrémité d'appui dotée d'une tête sphérique (20), et
un dispositif de support (10) à monter sur l'os du bassin (2) destiné à supporter l'extrémité d'appui proximale du clou médullaire de distraction (12a),
dans lequel le dispositif de support (10) comprend une embase (28) pouvant être fixée à l'os du bassin (2) et un dispositif de logement (18) agencé sur l'embase (28) pour recevoir de façon mobile l'extrémité d'appui du clou médullaire de distraction (12a), et le dispositif de logement (18) comprend un logement sphérique (48) comportant une cavité (58) formant une surface de logement sphérique destinée à recevoir la tête sphérique (20) disposée sur l'extrémité d'appui proximale, dans lequel le rayon intérieur de la surface de logement sphérique correspond au rayon extérieur de la tête sphérique (20) pouvant être logée et dans lequel le logement sphérique (48) est configuré de manière à ce que l'axe longitudinal (LT) du clou médullaire de distraction (12a) puisse se déplacer dans une région d'un cône imaginaire ayant un angle de cône de 25 à 45°, de préférence d'environ 35° et ne puisse pas se déboîter hors du logement sphérique (48) lorsque le clou médullaire de distraction (12a) est implanté dans l'os du fémur (4) et lorsque le dispositif de support (10) est monté sur l'os du bassin (2).

3. Dispositif selon l'une des revendications précédentes, dans lequel le clou médullaire de distraction (12, 12a) comprend à son extrémité d'appui une pièce intermédiaire (100) destinée à recevoir la tête sphérique (20).

4. Dispositif selon la revendication 3, dans lequel la pièce intermédiaire (100) comprend un rétrécissement (130) s'étendant sur son pourtour, qui est situé sur la pièce intermédiaire (100) immédiatement après la tête sphérique (20) vu de l'extrémité proximale.

5. Dispositif selon l'une des revendications précédentes, dans lequel au moins deux orifices de passage (74, 76) disposés à distance l'un de l'autre sont agencés dans l'embase (28) de façon distale par rapport au dispositif de logement (18).

6. Dispositif selon l'une des revendications précédentes, dans lequel le logement sphérique (48) comprend une base comportant une cavité partielle (84) dotée d'une première surface partielle de logement sphérique, qui est configurée et agencée de manière à ce que la tête sphérique (20) puisse être insérée librement dans la cavité partielle (84) et comprend une coiffe (72) comportant une seconde surface partielle de logement sphérique, qui peut être fixée de manière à ce que la première surface de logement sphérique se prolonge dans la seconde surface partielle de logement sphérique pour former une surface complète de logement sphérique, dans lequel la surface complète de logement sphérique est plus grande que la surface extérieure de la demi-tête sphérique (20).

7. Dispositif selon la revendication 6, dans lequel la surface complète de logement sphérique est de 5 à 10 % plus grande que la surface extérieure de la demi-tête sphérique.

8. Dispositif selon l'une des revendications précédentes, dans lequel l'embase (28) est aplatie à son bord proximal.

9. Dispositif selon l'une des revendications précédentes, dans lequel, pour diminuer le frottement de glissement, une coque en matière plastique (50) est insérée dans la cavité (58) ou un revêtement réduisant le frottement est appliqué sur la tête sphérique (20) ou la cavité est revêtue avec un matériau réduisant le frottement.
